# EUROPEAN PATENT APPLICATION

(11) **EP 4 588 363 A1**
(43) Date of publication of application: **23.07.2025**
(21) Application number: 24153136.7
(22) Date of filing: 22.01.2024
(51) Int. Cl.: A23L 31/00, C12N 1/20, C12N 13/00

(54) **APPLICATION OF ELECTRIC AND ELECTROMAGNETIC FIELDS IN SOLID STATE FERMENTATION TO MANUFACTURE EDIBLE MYCELIUM-BASED PRODUCTS**

(71) Applicant: Millow Holding AB, 421 30 Västra Frölunda (SE)
(72) Inventor: TAHERZADEH, Mohammad, 42244, Hisings Backa (SE); HELLWIG, Coralie, 41114, Göteborg (SE); TAHERZADEH, Esmaeil, 41114, Göteborg (SE); WAINAINA, Steven, 50741, Borås (SE)

(57) **Abstract**

**Technical Field**

The present invention relates to a method that applies electrical fields and/or static magnetic fields and/or extremely low electromagnetic fields to improve manufacturing of edible mycelium-based products. The said product comprises at least one fermented substrate and at least one strain of an edible non-mushroom filamentous fungus and/or in combination with edible bacteria in solid-state fermentation at high solid-loading conditions.

## Description

### Background of the invention

Electromagnetic fields (EMF) are known to affect living cells via several mechanisms. EMF can alter the rate of radical pair recombination. In fact, many reactions and in particular enzymatic ones contain rate determining steps in which two radicals are paired. Electromagnetic fields canalter stability of such pairs and so affect overall rate of the reactions (Grissom, Chemical Reviews, 1995, 95(1), 3-24). More than 200 papers reported the effect of EMF on free radicals (Levitt, et al., Reviews on Environmental Health, 2022, 37(1), 81-122). According to ion cyclotron equation, un-hydrated metal ions, e.g. Ca²⁺, absorb electromagnetic energy at few specific frequencies in the range of 50-60 Hz. This can be the reason for the well-known 'window' effect, i.e. response of living organisms to extremely low frequency EMF (Mahato, et al., LWT, 2023, 174, 114397). Another important influence of EMF on living organisms is through an effect on cell membrane function by various proposed mechanisms. The mechanisms include change in transmembrane voltage, altering Ca²⁺ distribution and local oscillation of ion concentration. In addition, another effect of extremely low frequency EMF on living organisms is by induction of an eddy current in culture media which may enhance diffusion-controlled mass transfer in stagnant layers close to the cell surface (Blank, Electromagnetic Biology and Medicine, 2008, 27(1), 3). EMF may also induce disruption and chain separation in DNA by acting on electrons in hydrogen bonds and weakening them (Blank, *Electromagnetic Biology and Medicine,* 2008, 27(1), 3). The static magnetic fields having intensities as large as 1 T may also rotate the DNA double helix around its axis and consequently tighten or loosen it depending on direction of rotation. The chain separation initiates at weakly bounded parts of the DNA (Blank and Goodman, Journal of Cellular Biochemistry, 2001, 81(4), 689-692). Pulsed electromagnetic fields at high intensities are known to induce cellular transcription (Goodman, et al., Science, 1983, 220:1283-1285). The threshold intensity for health effects of magnetic fields is reported to be greater than 50 mT (Blank and Goodman, Journal of Cellular Biochemistry, 2001, 81(4), 689-692).

The electric, magnetic, and electromagnetic fields are reported to affect fungal growth and morphology as well as their production of metabolites and enzymes. Growth of fungi proceeds by polarization of mycelium. Calcium influx through the channels at the tip of hyphae generates a calcium gradient in the hyphae. Transfer of constituents needed for construction and extension of the hyphae wall and membrane and also the cytoskeleton at the tip occurs in the hyphae along the gradient toward higher concentration of calcium ions. Calcium ion binds to calmodulin and the resulting complex activates calcineurin enzyme which removes phosphate from the transcription factor *Crzl.* Consequently, translocation is triggered thereby activating transcription of target genes encoding proteins involved in cell wall synthesis (Chen, et al., Current Fungal Infection Reports, 2010, 4, 244-255). Entrance of calcium ions into the hyphal tip is through two set of channels namely stretch gated channels and voltage gated channels. Stretch activated channels open when the tip is stretched by a barrier surface, and entered calcium ion cause the hyphae to change its grow direction to be parallel to the surface, a phenomenon which is known as "thigmotropism". Voltage gated channels open when an electrical field depolarize membrane and resulted calcium influx establishes cell polarity. This phenomenon is known as "galvanotropism". Visible galvanotropism is reported for some fungi whose hyphae are grown toward cathode (or anode or perpendicular to anode-cathode axis (Brand, et al. Current Biology, 2007, 17:347-352). Another concept, namely "magnetotropism", is used for plants and has recently been extended to microorganisms to represent enhancement of superficial growth rate of a yeast over a plate resulted from extremely low frequency magnetic fields of 60Hz and 3mT (Anaya, et al., Air Quality, Atmosphere & Health, 2023, 1-10).

While submerged fermentation using conventional fermenters is widely used in the production of fungal biomass and products, solid-state fermentation (SSF) is the natural habitat of filamentous fungi and is traditionally used in the production of koji, tempeh, blue cheese, among other products. Commercialization of SSF and widening of its applications has been attempted in recent years. Effect of electromagnetic fields on performance of filamentous fungi in submerged fermentation is reported in several papers. Application of static magnetic fields having intensities of 0.4mT and 1.6mT increased yield of red and yellow pigments. Using electromagnetic field at an intensity of 1 mT and frequency of 50Hz increased citric acid yield and cellulase activity of *Aspergillus. niger* proportional to exposure time (Jab 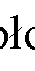 ońska, et al., *Advances in Applied Microbiology,* 2022, 121, 27-72). Some related patents applying submerged fermentation include, e.g., US20080182309A1 which revealed the use of static magnetic fields for production of lactic acid and ethanol using yeast, bacteria, or fungus. In another revelation, electrical fields and electromagnetic fields are applied on solutions containing cellulosic materials to produce fermentable sugars and enhance fermentation yield (US9410258B2).

Some scientific papers have reported the effect of EMF in SSF. Influence of static magnetic fields on production of protease by five fungal species showed that the North Pole significantly decreased the protease activity secreted by *Alternaria* sp., *Aspergillus niger,* and *Penicillium* sp. while the South Pole significantly increased the protease-specific activity of *Fusarium* sp. and *Mucor* sp. (Jabir and Sabah, International Journal of Advanced Research, 2017, 5(6), 2238-2245). Another paper demonstrated that a magnetic field having a low intensity of 7mT strongly affected inulinase production by *Geotrichum candidum* in solid state fermentation using leek as a potential carbon source (Canli and Kurbanoglu, Toxicology and Industrial Health, 2011, 28(10), 894-900). Patents reveal the application of an electromagnetic field in mushroom cultivation in the production of bioactive substances (CN108289422B) and in the assembly of a type of mushroom mycelium culture spot-punching machine (CN208908726U).

The present invention relates to a method that applies electrical fields and/or static magnetic fields and/or extremely low electromagnetic fields to improve manufacturing of edible mycelium-based products. The said product comprises at least one fermented substrate and at least one strain of an edible non-mushroom filamentous fungus and/or in combination with edible bacteria in solid-state fermentation at high solid-loading conditions.

### Summary of the invention

The method according to the present invention includes a step to prepare solid substrate that could be composed of a mixture of whole or crushed grains, brans, herbs and spices. The mixed solids are moistened by addition of appropriate amount of water to which some micro and macronutrients are dissolved optionally, and then inoculated with a fungus and prepared for solid-state fermentation in a bioreactor.

In another embodiment, the bioreactor is equipped with electrical voltage inducing and/or electromagnetic field producing equipment, such as Helmholtz coils or Merritt coils, that connect to the fermentation bed. According to another object of the present invention, during the fermentation course, intermittent electrical fields are established through the bed. Intensity of the electrical field up to 10 V per cm of the bed thickness are applied with durations from a few seconds to several minutes.

In another embodiment, intermittent low electromagnetic fields having intensities up to 10 mT and frequencies from 0, i.e., stagnant electromagnetic fields EMF, up to 100Hz for durations of a few seconds to several hours are applied through the bed during the fermentation course. The combination of electrical and electromagnetic fields with the above conditions could also be applied to the fermentation bed during the fermentation course.

### Detailed description of the invention

The basis of the present invention is based on the principle of balanced cooperation of various intracellular enzymes required during the growth of filamentous fungi. In this regard, there is a strong difference between submerged and solid-state fermentation, where many nutrients in SmF are soluble and readily absorbable into cells, while the substrate constituents such as carbohydrates, proteins and fats are mostly insoluble in SSF. Solubilizing of such compounds is performed by various hydrolyzing enzymes namely amylases, proteases, lipases, etc. Fungi are highly adapted for growth on moist solid substates with lower water activities, and are able to produce and exude various hydrolases. In SSF, the rate of substrate hydrolysis could be the fungal growth rate-determining step, and therefore any increase in appropriate extracellular hydrolyzing enzyme can increase the rate and extent of fungal growth. Extracellular enzymes serve other important functions, and might be the target product of SSF. They may improve digestibility or palatability of fermented substrate by consumers, release micronutrients, or produce bioactive compounds. Hence, these enzymes have crucial effect on fermentation performance.

According to the present invention, when an electromagnetic field is established in a bed of fermenting substrate, various changes may take place by different mechanisms in growing fungi. Electromagnetic fields loosen the hydrogen bonds and impose strain on DNA chains, and if the field has enough strength, the chains may separate. This process facilitates transcription enzymes to connect to the DNA chain and consequently boost gene transcriptions. It can lead to increased enzyme production and influence cell growth and other functions. DNA chain separation starts at locations with weaker bonds, and by increasing EMF strength, the locations at DNA with stronger bonds may also start to open, leading to expression of other genes. This may be one reason for strength dependency of electromagnetic field effects. Duration and extent of chain opening may be determined by duration of magnetic field pulse, i.e., its frequency. Along with other reasons, this may justify why only stagnant and extremely low frequency fields have observable effects on cell function.

In another embodiment, electromagnetic fields may increase specific activity of enzymes whose rate determining step include transient radical pairs. These pairs are stabilized by electromagnetic fields and so activation energy will be reduced which means enhanced enzyme specific activity. While effect of increased enzyme production remains after disappearance of electromagnetic field, the radical pair effect removes.

Accordingly, the invention reveals the integration of electrical and electromagnetic fields on the growing fungi based on the following concepts. To determine influence of electromagnetic fields on hydrolase secretion and activity as well as extent of growth, the following experiment was performed.

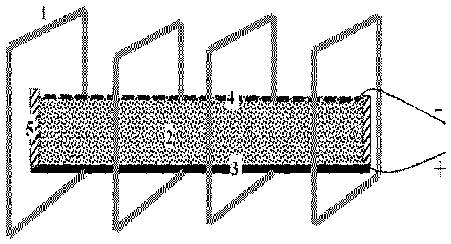

A SSF bioreactor was modified with a configuration revealed in Figure 1. The fermenting bed was surrounded by a Merritt coil system (1) having four-square coils. The coil system generates a uniform electromagnetic field of adjustable strength and frequency. It is connected to an appropriate electricity source with programmable AC intensity and frequency output. In special cases in which stagnant magnetic field is to be applied through the bed, the generator output is set to DC. The fermenting substrate (2) is loaded inside a tray (3) made from a non-ferromagnetic metal sheet. The frame of tray (5) is made from an electrical and thermal insulator material. In the same system bioreactor, electrical fields could also be applied to the bed, from upper surface to the fermenting bed bottom, or from side to side via non-ferromagnetic metal sheet electrodes (4). The bioreactor supplies sterile air stream with controlled flow rate, temperature, humidity and, if required, the other gases.

According to another object of the present invention, the generation of an electromagnetic field needs energy and generates excess heat, the extent of energy consumption rapidly increases as the size of coils are increased. Therefore, it was desirable to minimize energy consumption while keeping advantages of the electromagnetic field. This may be attainable by intermittently set the electromagnetic field on and off since, as said before, some electromagnetic field effects remain after the field removal. '

The impacts of electromagnetic and/or electric fields, according to the present invention, are assessed based on enzyme activity. For food-based applications, an additional sensory analysis was performed. Some examples are provided below.

### Example 1

Distillers dried grains with solubles (DDGS) is soaked in cold water for 20-120 minutes. Once dissolved, draining is performed such that only the absorbed water is present in the DDGS. The substrate should be slightly moist and crumply. Due to remaining moisture, no additional water needs to be added and the solid loading amounts to 60%.

The substrate is spread onto the special fermentation tray, acidic or alkaline agents are added prior to inoculation to adjust the pH value. First half of the edible filamentous fungu's spores, in this case *Neurospora intermedia,* is evenly stirred into the substrate, the second half is evenly sprinkled on top. The ratio between the edible filamentous fungus and the substrate is approximately 1:40

The temperature is set to 30 - 40°C, more preferably around 35 °C, pH of fermentation medium is set to 4.8, humidity is set to 85% Rh and air flow rate set at a minimum of 0.25 vvm. The airflow is adjusted automatically based on the CO2 concentration detected in the fermentation reactor. When the CO2 levels are in the range of 20000-30000 ppm, the installed sensors spontaneously attune the air flow rate to between 0.5-4 vvm. The substrate is fermented for at least 24 hours and more preferably for 40 to 50 hours.

Ann electromagnetic field at an intensity of 3mT and frequency of 60 Hz is stablished during thecourse of fermentation. Every 6 hours, 3 samples were taken from random locations of the bed. The resulting samples were divided into two sets. One set is extracted by adding 10 volume per weight water and shaking for one hour. The filtered extract was stored in refrigerator to be used to determine the activity of target enzymes. The other part was dried in oven, powdered, and used for determination of moisture content and also estimation of fungal growth extent via measurement of enzyme concentration. The fermentation product was harvested at hour 48 and organoleptic tests were performed on the product. As control, the same experiment was repeated at the same conditions in absence of the electromagnetic field.

Following standard methods, alpha amylase and xylanase activity of samples was measured. To estimate the contribution of the electromagnetic fields on enzyme reaction rates, a set of activity measurement for the mentioned enzymes was performed by putting the rack of test tubes containing enzyme-substrate solution onto the empty tray and establishing the same electromagnetic field, and then setting the air stream temperature to that of the enzyme assay conditions. The details are presented in table
1. As the results indicate, there was increased enzymatic activity when an electromagnetic field was applied. This indicated the possibility for enhancement of fermentation using this treatment.

**Table 1**

| **Fermentation time (h)** | **Alpha amylase (U/mg)** | | | **Xylanase (U/mg)** | | |
|---|---|---|---|---|---|---|
| | **a** | **b** | **c** | **a** | **b** | **c** |
| 6 | 0.01 ± 0.00 | 0.01 ± 0.00 | 0.02 ± 0.00 | 0.01 ± 0.00 | 0.01 ± 0.00 | 0.01 ± 0.00 |
| 12 | 0.01 ± 0.00 | 0.01 ± 0.00 | 0.05 ± 0.02 | 0.01 ± 0.00 | 0.01 ± 0.00 | 0.07 ± 0.01 |
| 18 | 0.02 ± 0.00 | 0.51 ± 0.27 | 0.15 ± 0.01 | 0.01 ± 0.00 | 1.06 ± 0.03 | 1.51 ± 0.04 |
| 24 | 0.22 ± 0.01 | 1.30 ± 0.43 | 1.50 ± 0.60 | 0.02 ± 0.01 | 2.46 ± 0.81 | 0.02 ± 0.01 |
| 30 | 0.35 ± 0.05 | 1.51 ± 0.66 | 1.81 ± 0.22 | 1.03 ± 0.02 | 2.03 ± 0.02 | 3.03 ± 0.05 |
| 36 | 0.42 ± 0.03 | 2.31 ± 0.15 | 2.01 ± 0.08 | 2.04 ± 0.07 | 3.97 ± 1.11 | 5.04 ± 1.08 |
| 48 | 1.43 ± 0.01 | 3.41 ± 0.70 | 4.51 ± 0.20 | 2.51 ± 0.02 | 5.00 ± 0.12 | 4.05 ± 0.03 |

| | | | | | | |
|---|---|---|---|---|---|---|
| *a: control, i.e. conventional solid-state fermentation* *b: conventional solid-state fermentation^ electromagnetic field treatment (continuous 3mT, 60Hz)* *c: enzyme activity assay for b samples was performed under the same electromagnetic field (continuous 3mT, 60Hz)* | | | | | | |

### Example 2

The conditions and procedures are the same as Example 1 but the field is turned on for 30 sec or 3 min at 1 or 3 hours intervals. *Rhizopus oryzae* was used as the inoculum and the solid loading was 55% and oat grains used as the substrate. Results of the cellulase activity as a result of the duration intervals of the applied electromagnetic fields are shown in Table 2.

**Table 2**

| **Duration and interval of electromagnetic applied field (3 mT & 60 Hz) conditions:** | **Cellulase content (U/mg)** |
|---|---|
| 30 sec duration - 1 hour interval | 0.08 ± 0.05 |
| 30 sec duration - 3 hours interval | 0.09 ± 0.01 |
| 3min duration - 1 hour interval | 0.08 ± 0.03 |
| 3 min duration - 3-hours interval | 0.12 ± 0.01 |

In addition to the aforementioned benefits on fermentation, the product organoleptic test was performed by a specialist panel using standard panel tasting methodologies. The scoring was based on a scale of 1 - 5 (5 = Like extremely, 4 = like, 3 = neither like nor dislike, 2 = dislike, 1 = dislike extremely). Referring to Table 3, there is a correlation between application of an electromagnetic field and consumer preference.

**Table 3**

| **Sample** | **Color** | **Aroma** | **taste** | **Texture** | **Overall acceptance** |
|---|---|---|---|---|---|
| Conventional | 4 | 3 | 3 | 3 | 3.25 |
| Electromagnetic field treated | 4 | 4 | 4 | 5 | 4.25 |

### Example 3

As stated in "background of invention", electrical fields may affect hyphal growth and orientation by opening of voltage gated channels which results in entrance of calcium ions into the hyphae tip which in turn will accelerate hyphal growth through several mechanisms. The effect of the electric field on acceleration of growth was experienced as described in this example.

The experiment's conditions were similar to Example 1 but no electromagnetic field was applied to the bed. Instead, a cover plate was fastened on the bed and connected to cathode electrodes of an electricity source. The floor plate was connected to anodes. An electrical field strength of 10 volt per centimeter of bed thickness was applied to the electrodes. A mixture of oat and beans (ratio 1:3 on wet basis) was used as substrate with a solid loading of 65% and *Rhizopus oligosporus* was used as the inoculum. Some experimental trials were performed. In the first experiment an electrical field was hold on continuously, while the duration and interval of field application were set differently, as shown in Table 4. The scoring was based on a scale of 1 - 5 (5 = Like extremely, 4 = like, 3 = neither like nor dislike, 2 = dislike, 1 = dislike extremely). Referring to Table 4, there is a correlation between the duration of applying electric field and consumer preference.

**Table 4**

| **Duration and interval of applied electric field (10 volts per centime of bed thickness) conditions:** | **Beta glucuronidase content (U/mg)** | **Overall acceptance** |
|---|---|---|
| 5 min duration - 1 hour interval | 3.58 ± 0.33 | 4.00 |
| 5 min duration - 3 hours interval | 4.33 ± 0.63 | 4.50 |
| 15 min duration - 1 hour interval | 4.82 ± 1.13 | 4.25 |
| 15 min duration - 3-hours interval | 3.88 ± 0.43 | 4.00 |
| Continuous | 4.98 ± 0.51 | 4.50 |

### Example 4

As stated in "background of invention", electrical fields may affect hyphal growth and orientation by opening of voltage gated channels which results in entrance of calcium ions into the hyphae tip which in turn will accelerate hyphal growth through several mechanisms. The effect of the electric field on acceleration of growth was experienced as described in this example.

The experiment's conditions were similar to Example 1 but no electromagnetic field was applied to the bed. Instead, a cover plate was fastened on the bed and connected to cathode electrodes of an electricity source. The floor plate was connected to anodes. An electrical field strength of 10 volt per centimeter of bed thickness was applied to the electrodes. Oat grains were used as substrate with a solid loading of 62%. In this trial, *Rhizopus oligosporus* and *Lactobacillus plantarum* were co-digested. Some experimental trials were performed. In the first experiment an electrical field was hold on continuously, while the duration and interval of field application were set differently, as shown in Table 5. The scoring was based on a scale of 1 - 5 (5 = Like extremely, 4 = like, 3 = neither like nor dislike, 2 = dislike, 1 = dislike extremely). Referring to Table 5, there is a correlation between the duration of applying electric field and consumer preference.

**Table 5**

| **Duration and interval of applied electric field (10 volts per centime of bed thickness) conditions:** | **Beta glucuronidase content (U/mg)** | **Overall acceptance** |
|---|---|---|
| 5 min duration - 1 hour interval | 3.48 ± 0.73 | 4.25 |
| 5 min duration - 3 hours interval | 4.43 ± 0.71 | 4.50 |
| 15 min duration - 1 hour interval | 4.72 ± 0.16 | 4.25 |
| 15 min duration - 3-hours interval | 3.89 ± 0.63 | 4.25 |
| Continuous | 5.25 ± 0.41 | 4.50 |

### Example 5

It is visible from the previous examples that both electromagnetic and electrical fields have positive effects on the performance of solid state fermentation. Therefore, it is justifiable to indentify the combined effect of electrical and electromagnetic fields. In this example, the experimental conditions were similar to Example 1 and the first experiment was performed as control and with no electrical and electromagnetic field. The second experiment was run with intermittent electrical and electromagnetic fields. The electromagnetic fields were applied for 3 min and 3 hours intervals from onset of fermentation. *Aspergillus oryzae* was used as the inoculum and the solid loading was 55%. The electric field was applied in 10 min durations and 3 hours intervals and started 2 hours after the fermentation started (Table 6). The scoring was based on a scale of 1 - 5 (5 = Like extremely, 4 = like, 3 = neither like nor dislike, 2 = dislike, 1 = dislike extremely). Referring to Table 6, there is a correlation between the duration of applying an electric field and consumer preference.

**Table 6**

| **Fermentation conditions** | **Alpha amylase content (U/mg)** | **Overall acceptance** |
|---|---|---|
| Conventional | 4.51 ± 0.20 | 4.50 |
| Applied electric field of 10V/cm by 15 min duration at 3 hours intervals + 3 min electromagnetic field of 3mT&60Hz at 3 hours intervals. | 4.55 ± 0.03 | 4.50 |

The above examples are intended to serve as illustrations but not as limitations of the present invention. For food applications, the substrate may be varied to include other grains such as, but not limited to, wheat, rye and barley, or legumes such as, but not limited to, peas, beans and lentils, as well as vegetables such as, but not limited to, potatoes, carrots and beets. Furthermore, industrial residues such as, but not limited to, DDGS, brewery spent grains (BSG) and bread leftovers can be used, particularly for feed applications. The term 'food' applies to nutrition source for humans whereas the term `feed' applies to nutrition source for animals, e.g. ruminants, pets and aquatic creatures like fish.

It is also conceivable that the methods revealed in this invention can be applied for production of a variety of other valuable mycelium-based products such as, but not limited to, packaging materials and textiles.

## Claims

1. A method for enhancing growth of at least one strain of an edible fungus on at least one substrate in solid state fermentation by applying electric fields, and/or stagnant or extremely low frequency electromagnetic field during the course of fermentation.
a) wherein the edible filamentous fungi comprise non-mushroom strains, consisting of strains such as *Mucor indicus, Fusarium venenatum, Neurospora intermedia, Rhizopus oligosporus, Aspergillus oryzae* or *Rhizopus oryzae* or a mixture thereof; and
b) wherein the solid state fermentation is performed at solid loading conditions of above 50%.

2. A method according to Claim 1 in which edible bacteria is co-cultivated with edible filamentous fungi; wherein the edible bacteria comprise of strains such as *Lactobacillus plantarum, Lactobacillus fermentum, Bifidobacterium infantis* or *Bifidobacterium longum* or a mixture thereof.

3. The method according to Claim 1 in which the said electromagnetic field is generated by Merritt coil, Helmholtz coil, or solenoid.

4. The method according to Claim 1 wherein the said electromagnetic field has a frequency of 10 - 100 Hz, preferably 50 - 60 Hz.

5. The method according to Claim 1 wherein the said electromagnetic field has a strength of 0.1mT - 50mT preferably 0.5 -10 mT.

6. The method according to Claim 1 wherein the said electromagnetic field has a duration of 0.1 - 60 min preferably 5-30 min.

7. The method according to Claim 5 wherein the said electromagnetic field is repeated every 2 - 20 hour preferably 5 - 15h.

8. A method according to Claim 1 in which electric field strength is in range 1 - 50 V and in particular 10V per centimeter of the fermenting bed.

9. A method according to Claim 1 in which electric field has a duration of 1 - 20 min.

10. A method according to Claim 1 in which the said electric field is imposed every 1 - 20 h preferably every 5 - 15h

11. A food product made according to the proceeding Claims 1 - 10.

12. A feed product made according to the proceeding Claims 1 - 10.
